# EUROPEAN PATENT SPECIFICATION

(11) **EP 1 263 330 B1**
(45) Date of publication and mention of the grant of the patent: **18.06.2014**
(21) Application number: 01918427.4
(22) Date of filing: 05.03.2001
(51) Int. Cl.: A61B 17/10

(54) **SUTURE CLIP DELIVERY DEVICE**
VORRICHTUNG ZUR ANBRINGUNG VON NAHTKLAMMERN
DISPOSITIF D'APPLICATION DES ELEMENTS DE BLOCAGE DE SUTURE

(30) Priority: 03.03.2000 US 186926 P; 19.05.2000 US 205741 P; 19.05.2000 US 205444 P; 29.11.2000 US 253970 P
(43) Date of publication of application: 11.12.2002
(60) Divisional application: 10180078.7
(73) Proprietor: C.R. BARD, INC., Murray Hill New Jersey 07974 (US)
(72) Inventor: GAMBALE, Richard, A., Tyngsboro, MA 01879 (US); WEISER, Michael, F., Groton, MA 01450 (US); PAGE, Edward, Carlton, Baldwinville, MA 01436 (US); KOLNICK, Alexander, Marblehead, MA 01945 (US)
(74) Representative: Grey, Ian Michael
(86) International application number: PCT/US2001/007349
(87) International publication number: WO 2001/066001

(56) References cited:
- WO-A-97/30639
- WO-A-99/04698
- JP-A- 7 136 177
- US-A- 5 199 566
- US-A- 5 514 159
- US-A- 5 899 921
- US-A- 5 931 844
- US-A- 6 001 110
- US-A- 6 126 677

## Description

The disclosed invention relates generally to devices used to secure sutures.

More particularly, the invention relates to suture clips and suture clip delivery devices used in conjunction with sewing devices used in flexible endoscopy; though it is also applicable to devices used in rigid endoscopy.

It is estimated that as many as 15,000,000 individuals in the United States suffer from stomach acid reflux into the lower esophageal region, commonly referred to as GERD (gastroesophageal reflux disorder). Although the illness may result from a wide variety of causes, it is ultimately the failure of the cardiac sphincter located above the stomach that enables a reflux event to occur. A surgical method developed to reduce reflux episodes involves forming tissue folds in the walls of the stomach to reduce the cross-sectional area of the gastroesophageal juncture to mimic the function of the cardiac sphincter. To perform these types of procedures, sewing devices used to suture the stomach wall into folds are used. The procedure typically involves a fiber optic endoscope introduced into the lower esophageal area. A sewing instrument is advanced down the working channel of the endoscope that has an aspiration port for generating negative pressure to suction stomach wall tissue into the sewing instrument where one or more sutures are implanted to hold the suctioned tissue in a folded condition known as a plication.

Sewing devices for this procedure are described in, for example, GB-A-2165559 and U.S. Pat. No. 5,080,663. According to these references, a sewing device is used for passing a thread through a tissue fold. The sewing device comprises a hollow needle movable between a first pre-tissue penetration position and a second position in which it passes through the tissue, with a thread carrier adapted to be attached to the thread and being receivable within the hollow needle.

Preferably, the sewing device comprises a body that defines a cavity within which the substrate portion can be held, for example, by means of suction. The hollow needle is mounted for movement in the body between the first and second positions. In some versions of the procedure, a suture is inserted into the tissue with a needle, the two ends of which are fed back out of the patient. Typically, a physician fashioned a series of half hitches to secure the suture to the subject tissue.

In other embodiments of the procedure, a tag attached to a distal end of the suture and contained within a lumen of the needle is inserted into and past the tissue. With the needle in an advanced position, i.e., with the needle distal tip extending distally beyond the pierced tissue, a pusher forces the tag out of the needle. After retraction of the needle from the tissue, the suture is retracted so that the tag contacts the tissue. The tag functions as an anchor that enables the suture to be secured to the tissue from the proximal end and also disperses the force applied to the tissue by the suture to prevent tearing of the tissue. Such a device and procedure is described in U.S. Patent No. 5,080,663 to Mills et al., the contents of which are incorporated herein by reference.

One of the significant problems associated with these procedures is the time and number of intubations needed to perform the various procedures endoscopically. Due to a number of concerns, a patient is typically anesthetized for no more than approximately 40 minutes. In this period of time, procedures such as the GERD procedure must be performed to completion. In the GERD procedure, several intubations are performed to create several plications. As many as nine intubations are required to create just one plication. This is the case when half hitches are used to secure the suture. Each half hitch requires the hitch to be made outside the endoscope and then advanced down the endoscope with a pusher. Typically, six half hitches are used per suture thus six intubations are needed to secure the half hitches. The time needed for each intubation substantially reduces the working time to complete a GERD procedure.

One approach to solving this problem is disclosed in U.S. Patent No. 5,584,861 to Swain. The Swain patent discloses a suture clip and suture clip delivery device that is used in place of half hitch knots. The disclosed suture clip is a cylinder with a plug that can be releasably secured in the cylinder. The disclosed suture clip delivery device includes a tube, the distal end of which has a recess for receiving the suture clip. An axially movable stirrup is provided at the distal end that has the capacity to be moved from a first position that secures the suture clip to the tube and a second position that allows for the suture clip to be removed from the recess.

An aperture is provided in the cylinder to receive the suture. The cylinder is advanced over the suture that exits from a proximal end of the cylinder and enters the tube. An aperture in a sidewall of the tube provides egress for the suture. The plug is then advanced down the tube and into the cylinder. The interfacing walls of the cylinder and plug capture the suture. A pusher is used to force the plug into the cylinder while the stirrup maintains the suture clip in the recess. Following plug insertion, the stirrup, which is offset from the center axis of the tube, is advanced distally from the distal end of the tube to release the suture clip from the tube.

Although the Swain device solves the problem of multiple half hitches, the overall design of the device has certain drawbacks. First, to successfully join the cylinder to the plug to form the suture clip, the stirrup must be physically maintained in a retracted position while an opposing force is applied with a pusher to the plug. Second, the presence of the stirrup inevitably prevents the tube and therefore, the suture clip from being placed tight against the sutured tissue. This opens the possibility for slack to develop between the clip and the tissue, which can potentially lead to a relaxation of the desired tissue fold.

Suture anchors or clips and the means to deliver and secure them are quite common in the medical industry as they play a significant role in simplifying the tedious task of securing tissue previously accomplished by tying knots on sutures. Quite common are metallic twist tie, staples and various forms of plastic or metallic permanent or temporary mechanical means to prevent the suture from slipping through the tissue. As a result of their function, the clips are typically designed to be large to overcome the stresses expected of them. Disclosed are several single and multi-component suture clips as well as a variety of relatively simple compact suture clip delivery devices that can be inserted into a natural body orifice or through the working channel of an endoscope to cinch a suture clip into the desired position in close proximity to or against the plication.

It is to be appreciated that the suture clips and suture clip delivery devices disclosed herein have a potentially wide range of applications including, but not limited to, the attachment of devices, e.g., pH monitor to the gastrointestinal wall, the closure of perforations or ulcers and the creation of anastomoses. Another useful application involves the use of radiopaque clips as fluoroscopic markers.

A drive tool for delivering a suture securing device to a surgical site is known from US 5931844.

A suture clip delivery catheter according to the invention is characterised by the features recited in the characterising portion of claim 1.

One of the suture clip delivery and locking systems described herein includes a tool designed to be attached to the distal end of an endoscope or catheter among other possibilities. The tool has a body from which finger-like segments project distally. The finger-like projections are made of a material that allows the finger-like projections to flex or spring from a first closed position to a second open position and back to the first position. The finger-like projections define a chamber within which a suture clip is premounted or introduced by being advanced through the endoscope or catheter. The chamber is defined axially at a distal end by tangs extending radially inwardly from the distal ends of the finger-like projections and at a proximal end by proximal tangs or cam followers that extend radially inwardly from inner walls of the finger-like projections. A pusher, adapted to slide within the tool's body, is provided having a head that is adapted to mate with the cam follower to move the finger-like projections to the second open position when the pusher is advanced distally in and through the endoscope or catheter.

The suture clips described herein are designed to allow the suture to interwind through the clips in such a manner that the clips move with minimal friction while in an open position. In a closed position, the clip captures the suture, by the increased friction. The suture passes proximally through the chamber between the pusher head and mating tang and then outward through a lumen in the chamber and continues proximally outside the endoscope or catheter to the proximal end of the entire system. The user may thread a suture through a clip and then load the clip into the tooling. The clip may, in certain designs, already be premounted and then require the final advance by the physician to the site. At the site the physician activates the handle to apply a force to the clip, thereby locking it to the suture. The application of force first secures the clip and captures the suture material within the mating surfaces, and then expels the clip from the tool to remain within the patient.

The present invention pertains to improvements to an endoscopic suturing device such as that disclosed in U.S. patent nos. 5,792,153 and 5,080,663. The improved suturing device of the present application can be used to suture tissue internally via an endoscope for a wide variety of purposes such as: attaching a feeding tube to small intestine; closing intestinal openings in the case of a fistula, repairing esophageal tears and suturing tissue sites of localized bleeding. However, the invention is especially useful in the endoscopic suturing procedures to treat gastroesophageal reflex disease (GERD).

Another embodiment of the suture delivering, locking and severing systems described herein includes a multi-coaxial catheter with a three or four finger collet jaw affixed at a distal end. The catheter has a distal end from which the collect fingers distally project. The collet fingers are made of a material such as stainless steel or an engineering grade of plastic that allow the collet fingers to flex or spring from a pre-biased first open position to a second closed position and back to the first position. Alternatively, the collet fingers can be designed to flex or spring from a pre-biased first closed position to a second open position and back to the first position. The collet fingers define a cage within which a suture clip assembly is premounted. The cage is defined proximally by a distal end of the collet body and distally by flanges extending radially inwardly from the distal ends of the collet fingers. The case is sized so that a plurality of suture clip plugs can be preloaded into the cage along with a single suture clip ring. By biasing the collet fingers in an open position, the need for cam surfaces and cam followers is eliminated.

With any of the embodiments, the outer sliding sleeve is provided to secure the collet fingers in a closed position when placed in a distally advanced position. The outer sliding sleeve performs the additional function of severing the suture at a point proximal to the suture clip after engagement of the suture upon proximal retraction. One or more sutures slot are provided in the distal end of the outer sliding sleeve to provide suture exits. A distal end of the suture slots are sharpened to sever the suture. Upon proximal retraction of the outer sliding sleeve, the distal end of suture slot engages the suture and severs it when the distal end of the suture slot travels proximally to the proximal end of the collet fingers. Optionally, a fixed metallic ring can be affixed to the outer surface of the collet at a point proximal to the collet fingers and inside the outer sliding sleeve. The metallic ring is formed with a sharp distal tip that engages and severs the suture when the outer sliding sleeve is proximally retracted. The design enables the suture clip to be cinched in close proximity to the sutured tissue as well as allow for the severing of the suture tails and release of the suture from the delivery device in one step.

The suture clips designed for use with the referenced suture clip delivery systems are comprised of a plug and a ring that are configured to allow a suture to interwind through the clip in such a manner that the clip components move with minimal friction while in an open position. The plug is a headless design that has features that allow for a positive lock with the ring. Channels are provided in the plug to provide access ways for the suture to lessen the effort needed to thread the suture into the plug. The locking features of the plug are compressible so that when in a locked state, the suture is captured via a combination of frictional engagement and the locking surfaces The plugs have features built into their proximal and distal ends to allow stacking of the plugs to enhance alignment for the delivery of axial forces to set the plugs in rings. Additionally, a diverter for channeling the suture into axial slots formed in the plug is provided in one embodiment to extend distally of the distal end of the ring, when assembled with the plug, to interrogate the tissue and cause fibrosis. The fibrosis causes the tissue to become more bulky which is though to enhance the therapeutic effect of this technique.

In another embodiment, the plug and ring are formed with inter-locking ribs or scales that enhance the advancement of the plug into the ring and prevent disengagement. The ribs are fashioned to allow one-way movement of the suture through the suture clip.

Once threaded into the ring and plug, the suture is passed through finger slots formed between adjacent collet fingers and out the suture slot of the outer sliding sleeve. This enables the suture tails to be channeled externally of the catheter for removal at the proximal end of the catheter outside the patient after the tails have been severed at a point proximal to the now assembled suture clip. The catheter operator may thread a suture through a clip and then load the clip into the tooling or thread the suture through a premounted clip. At the site the physician activates the handle to apply a force to the clip, thereby locking it to the suture. The application of force would first secure the clip components capturing the suture material within the mating surfaces, and then expel the clip from the tool to remain within the patient.

Suture clips disclosed herein include combination friction fit and positive locking embodiments where the components of the clips capture the suture with friction and lock the suture in place with inter-locking surfaces. It is to be appreciated that a wide variety of suture clip configurations can be formed from the basic ring/plug configuration that employs a friction fit/positive lock securing means.

A yet further suture locking and severing system described herein includes, in one general embodiment, a multi-coaxial catheter, and in a second general embodiment, a system dimensioned for use in the working channel of an endoscope. Each general embodiment has a two, three or four finger collet jaw affixed at a distal end. The collet jaw has a collet body from which the collet fingers distally project. The collet fingers are made of a material such as stainless steel or high modulus plastics that allow the collet fingers to flex or spring from a first closed position to a second open position and back to the first position. Alternatively, the collet fingers can be designed to flex or spring from a first open position to a second closed position and back to the first position. This is accomplished by providing a radial bias in either the open or closed position. The collet fingers define a cage within which a suture clip assembly is premounted. The cage is defined proximally by a distal end of the collet body or by ramps formed on the inside walls of the collet fingers distal to the collet body and distally by flanges extending radially inwardly from the distal ends of the collet fingers. A further spatial restriction is provided toward the proximal end of the cage by the ramps that extend radially inwardly from the inner walls of the collet fingers and that additionally function as cams to open the collet fingers.

A two-pusher system is employed that utilizes an inner pusher to secure a plug to a ring that together comprise the suture clip. A second pusher provided coaxially about, and in sliding engagement with, the inner pusher has a tapered distal end that interacts with the proximal ramps when advanced distally to cause the collet fingers to move from a closed position to an open position.

An outer sliding sleeve can be provided to secure the collet fingers in a closed position when placed in a distally advanced position. The outer sliding sleeve performs the additional function of severing the suture at a point proximal to the suture clip after engagement of the suture. A suture slot is provided in the distal end of the outer sliding sleeve to provide a suture exit. Upon proximal retraction of the outer sliding sleeve, the distal end of the suture slot engages the suture and severs it when the distal end of the suture slot travels proximal to the proximal end of the collet fingers. Optionally, a fixed metallic ring can be affixed to the outer surface of the collet at a point proximal to the collet fingers and inside the outer sliding sleeve. The metallic ring is formed with a sharp distal tip that engages and severs the suture when the outer sliding sleeve is proximally retracted. The design enables the suture clip to be cinched in close proximity to the sutured tissue as well as allow for the severing of the suture tails and release of the suture from the delivery device in one step.

The suture clips designed for use with the suture clip delivery system are comprised of a plug and a ring that are configured to allow a suture to inter-wind through the clip in such a manner that the clips move with minimal friction while in an open position. In a closed position, the clip captures the suture by frictional engagement. Once threaded into the ring and plug, the suture is passed through finger slots formed between adjacent collet fingers and out the suture slot of the outer sliding sleeve. This enables the suture tails to be channeled externally of the catheter for removal at the proximal end of the catheter outside the patient after the tails have been severed at a point proximal to the now assembled suture clip. The catheter operator may thread a suture through a clip and then load the clip into the tooling or thread the suture through a premounted clip. After positioning the delivery system at the sutured tissue site, the device operator activates the handle to apply a force to the clip, thereby locking it to the suture. The application of force first secures the clip components thus capturing the suture material within the mating surfaces, and second expels the clip from the delivery system tool to remain within the patient.

Suture clips disclosed herein include friction fit embodiments where the components of the clips capture the suture with friction. It is to be appreciated that a wide variety of suture clip configurations can be formed from the basic ring/plug configuration that employs a friction fit securing means. Of course, the ring and plug components of the clip can be provided with interlocking features for enhancing the suture capturing effect.

In a still further embodiment, a suture clip delivery device having pivoting collet fingers is disclosed. The collet fingers rotate about a pin secured to a collet cage body. A pusher, suture clip component or other component radially restrains the pivoting collet fingers from pivoting radially outwardly at a distal end when proximal to a cinched position. Another embodiment employs a ring secured about the collet cage body. A distal edge of the ring provides a pivot point and eliminates the need to secure the pivoting collet fingers to the collet cage with pins.

Also disclosed is a suture clip loader used to deliver the suture clip components into the collet cage. The suture clip loader has two main components, a main body through which a hypotube is secured and a plunger comprising a plunger head and a plunder rod. The plunger rod is dimensioned to slide freely within the hypotube. A suture clip ring is placed over a first end of the hypotube that is situated within a cavity formed in the loader main body. The distal end of a suture clip plug is loosely fit within the lumen of the first end for delivery into a collet cage. A collet cage with the collet fingers in an open position is advanced over the plug, hypotube and ring. Advancement of the plunger into the hypotube from a second hypotube end causes disengagement of the suture clip plug into the collet cage. The collet cage fingers are then moved into a closed position by advancing the outer sliding sleeve so as to grasp the suture clip ring that is retained in the collect cage when the collet cage is removed from the hypotube.
Figure 1 is a side perspective view of a suture clip plug for use with the suture clip delivery catheter according to one embodiment of the invention;
Figure 2 is a bottom perspective view of the suture clip plug according to one embodiment of the invention;
Figure 3 is a side sectional perspective view of the suture clip plug shown in Figure 1;
Figure 4 is a side perspective view of the suture clip of Figure 1 assembled to a suture;
Figure 5 is a side sectional view of an alternative suture clip assembly with rib formations for use with the suture clip delivery catheter of the invention;
Figure 6 is a partial cutaway side perspective view of a double pusher suture clip locking and severing catheter distal end with a pre-mounted suture clip assembly according to one embodiment of the invention;
Figure 7 is a partial sectional front perspective view of a double pusher suture clip locking and severing catheter distal end with a pre-mounted suture clip assembly according to one embodiment of the invention;
Figure 8 is a partial sectional top perspective view of a double pusher suture clip locking and severing catheter distal end with an outer sliding sleeve partially retracted according to one embodiment of the invention;
Figure 9 is a partial sectional front perspective view of a double pusher suture clip locking and severing catheter distal end according another embodiment of the invention;
Figure 10 is back perspective view of a double pusher suture clip locking and severing catheter distal end and partial hypotube body according to one embodiment of the invention;
Figure 11 is a side perspective view of a double pusher suture clip locking and severing catheter according to one embodiment of the invention;
Figure 12 is a partial cutaway side perspective view of a double pusher suture clip locking and severing catheter distal end with loaded and threaded suture clip components according to one embodiment of the invention;
Figure 13 is a side sectional view of a double pusher suture clip locking and severing catheter distal end with pre-loaded suture clip components according to one embodiment of the invention;
Figure 14 is a side sectional view of a double pusher suture clip locking and severing catheter distal end with an inner pusher in an advanced position and a suture clip in a cinched condition according to one embodiment of the invention;
Figure 15 is a side sectional view of a double pusher suture clip locking and severing catheter distal end with an outer sliding sleeve in a partially retracted position according to one embodiment of the invention;
Figure 16 is a side sectional view of a double pusher suture clip locking and severing catheter distal end with an outer sliding sleeve in a fully retracted position according to one embodiment of the invention;
Figure 17 is a side sectional view of a double pusher suture clip locking and severing catheter distal end with an outer pusher in an advanced position engaging and opening collet fingers according to one embodiment of the invention;
Figure 18 is a side sectional view of a double pusher suture clip locking and severing catheter distal end with a suture clip being released from a collet cage according to one embodiment of the invention;
Figure 19 is a top perspective view of another assembled suture lock plug and suture lock ring for use with the suture clip delivery catheter according to an embodiment of the invention;
Figure 20 is a sectional view of another assembled suture lock plug and suture lock ring for use with the suture clip delivery catheter according to an embodiment of the invention;
Figure 21 is an end view of the assembled suture lock plug and suture lock ring shown in Figure 20;
Figure 22 is a perspective view of the suture lock plug and suture lock ring shown in Figure 20, unassembled;
Figure 23 is a perspective view of a suture lock plug for use with the suture clip delivery catheter according to an embodiment of the invention;
Figure 24 is a side sectional view of a suture clip loading device for use with the suture clip delivery catheter according to an embodiment of the invention;
Figure 25 is an end view of the suture clip loading device shown in Figure 24;
Figure 26 is a perspective view of the suture clip loading device shown in Figure 24;

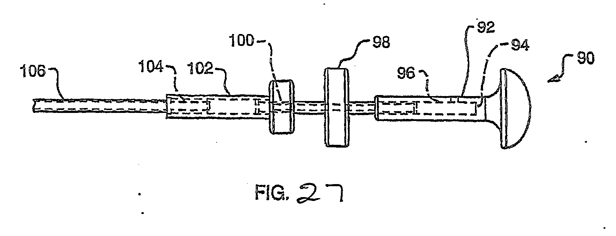

Figure 27 is a side sectional view of a control handle for use with the suture clip delivery catheter according to an embodiment of the invention;
Figure 28 is a side sectional view of the control handle shown in Figure 27;
Figure 29 is a plan view of a single action control handle for use with the suture delivery catheter of the invention;
Figure 30 is a sectional view of a single action control handle for use with the suture delivery catheter of the invention;
Figure 31 is a partial sectional view of a single action control handle for use with the suture delivery catheter of the invention;
Figure 32 is a partial sectional view of a single action control handle for use with the suture delivery catheter of the invention;
Figure 33 is a side sectional view of a threader for use with the suture delivery catheter according to the invention;
Figure 34 is an end view of the threader shown in Figure 33;
Figure 35 is a perspective view of the threader shown in Figure 33;
Figure 36 is a side sectional view of a vacuum-actuated threader for use with the suture delivery catheter according to the invention;
Figure 37 is a front elevational view of the vacuum-actuated threader shown in Figure 36;
Figure 38 is a side sectional view of the vacuum-actuated threader shown in Figure 36;
Figure 39 is a front elevational view of the vacuum-actuated threader shown in Figure 36.

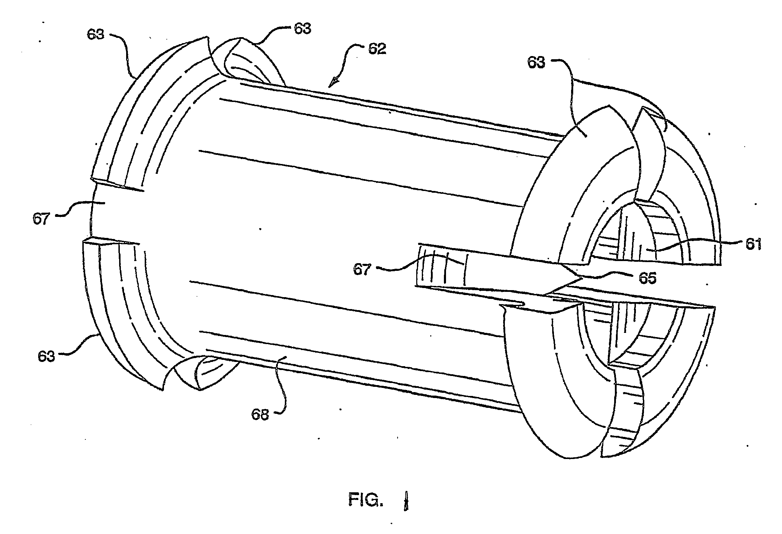

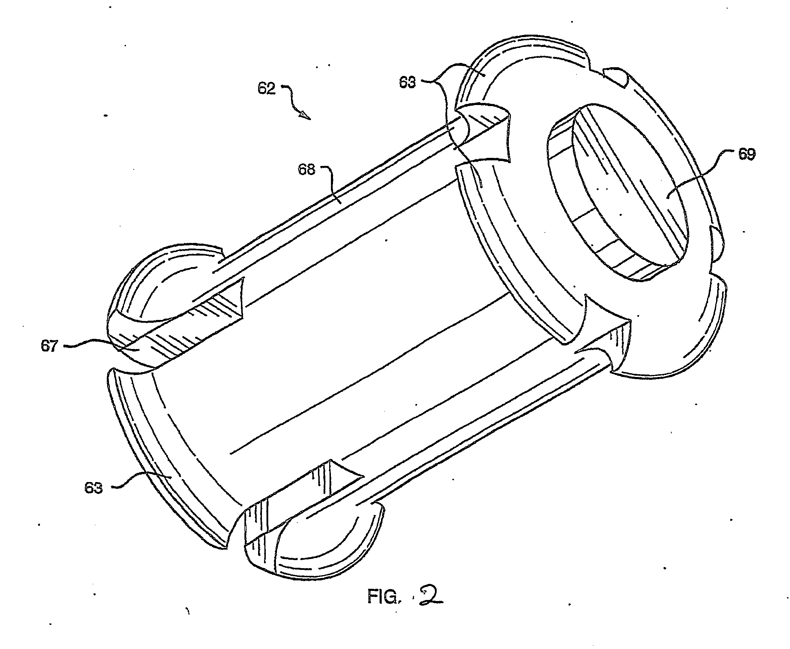

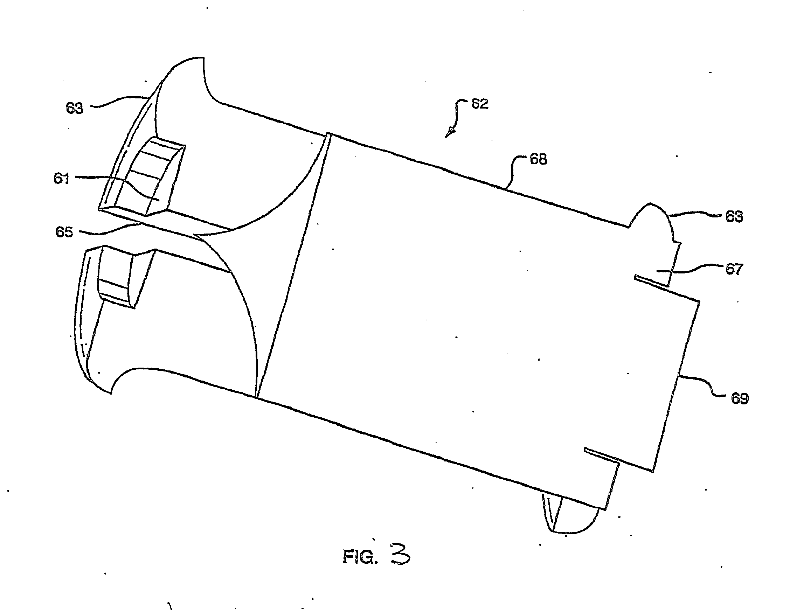

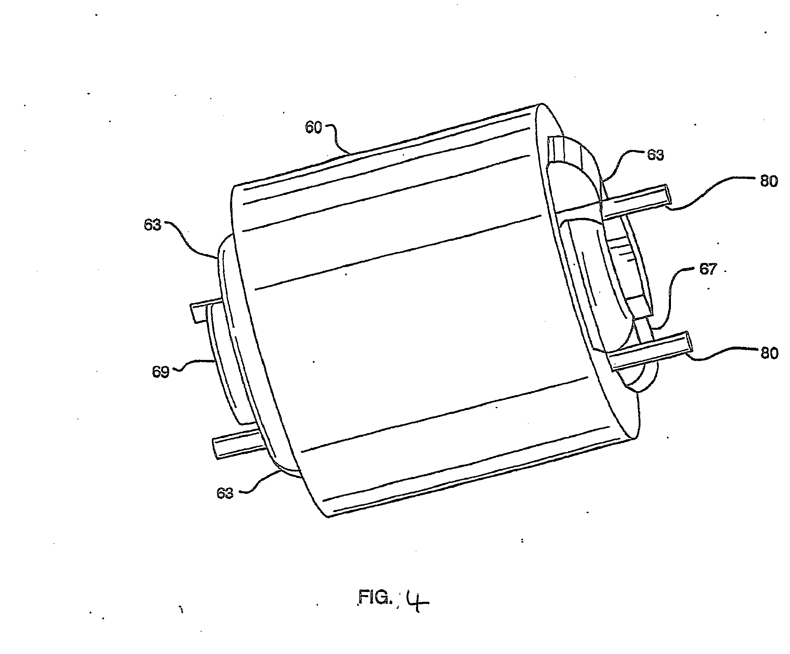

The suture clips used with the suture clip delivery catheter described herein have plugs without heads. As shown in Figures 1 to 3, plug 62 that is preferably injection molded, has a main shaft 68 that is adapted to frictionally engage the inner walls of ring 60. Extending from the distal and proximal ends of plug 62 are a plurality of plug locking tabs 63 which have outer faces that are radiused about the edges to provide ease of advancement into ring 60 which is also preferably injection molded. Locking tabs 63 are formed with sufficient flexibility to distort to ease advancement into ring so that their overall diameter is reduced while traveling through ring 60. Once the distal most locking tabs emerge from the distal end of ring 60, preferably simultaneous with the contact of the proximal locking tabs 63 with the proximal end of ring 60, the distal locking tabs 63 spring back to their original radially expanded state. The diameter of the flanges is set to be greater than the outside diameter of ring 60 so that when fully radially expanded, locking tabs 63 situated on the proximal and distal ends of plug 62 cooperate to positively lock ring 60 in an axial direction as shown in Figure 4.

To reduce the effort needed to advance suture 80 about plug 60, plug guide slots 67 are formed between the side surfaces of locking tabs 63. A central diverter 65 is formed extending from the central distal end of plug 60. Diverter 65 has tapered sidewalls that increase radially outwardly from a distal to a proximal end. This configuration facilitates tracking and the radial disposition of suture 80 into guide slots 67. In one embodiment, diverter 65 extends distally beyond the distal end of plug 62 and an engaged ring 60 so that when the assembled suture clip is appended to suture 80, diverter 65 contacts the sutured tissue and causes fibrosis which leads to a thickening of the tissue. It is believed that this enhances the therapeutic effect of the procedure in GERD patients.

To permit stacking of multiple plugs 62, a substantially cylindrical plug cavity 61 is formed on the distal end of plug 60 and a corresponding axially extending cylindrical plug projection 69 is formed in the proximal end. To ensure positive engagement, plug projection 69 is extended above plug locking tabs 63. Plug cavity 61 and plug projection 69 are sized to loosely mate when a plug cavity 61 in the distal end of one plug is aligned with a plug projection 69 of an adjacent plug. The size tolerances for the respective mating components are maintained sufficiently loose not to interfere with suture clip deployment but tight enough to provide axial and radial alignment during the application of compressive forces to join a plug to a ring in the delivering and locking catheter described herein.

In practice, diverter 65 feeds suture 80 into guide slot 67 that is sized to allow the free movement of plug 60 along suture 80. This configuration provides a suture clip plug that decreases the effort needed to advance the plug over the ends of a suture before loading into the suture clip delivering and locking device.

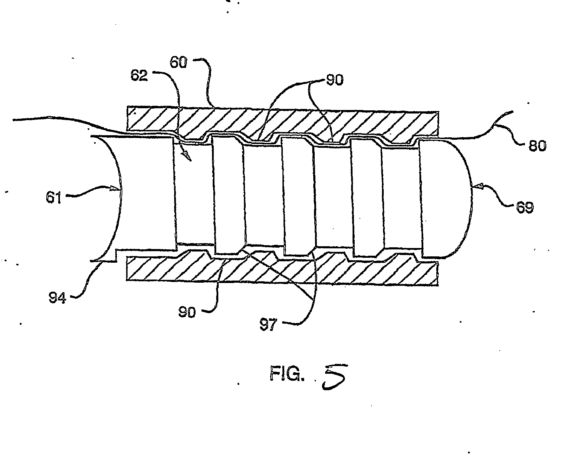

A further suture clip embodiment employs rings and plugs with interlocking ribs or scales. As shown in Figure 5, ribs 90 are provided circumferentially about the inner wall of ring 60. Corresponding plug ribs 97 are provided about the outer sidewall of plug 62. The ribs are configured to allow for the advancement of the plug into the ring with minimal effort. The ribs are tapered to allow the plug to be inserted into the ring in a distal direction and create an interference that prevents or resists proximal retraction of plug 62 out of ring 60. The suture 80 becomes entrapped between the inter-engaging ribs. The depth of the scale feature as well as the angle or taper is dependent on the suture size, material and degree of locking needed such as the life expectancy of the individual. Significant profiles will provide higher holding forces that can also adversely affect the life expectancy of the captured suture. The hardness of the components also should be factored into the design. The profile selected is preferably easily molded on the plug with the mold pulling off in the correct fashion. The inner ring detail becomes less of a challenge with the use of a spiral rib that allows the mold pin to be unthreaded. Optionally, a proximal stop 94 can be employed to limit distal advancement of the plug absent plug locking tabs 63. In the embodiment shown in Figure 5, plug projection 69 and plug cavity 61 are shown having mating convex and concave domed surfaces, respectively.

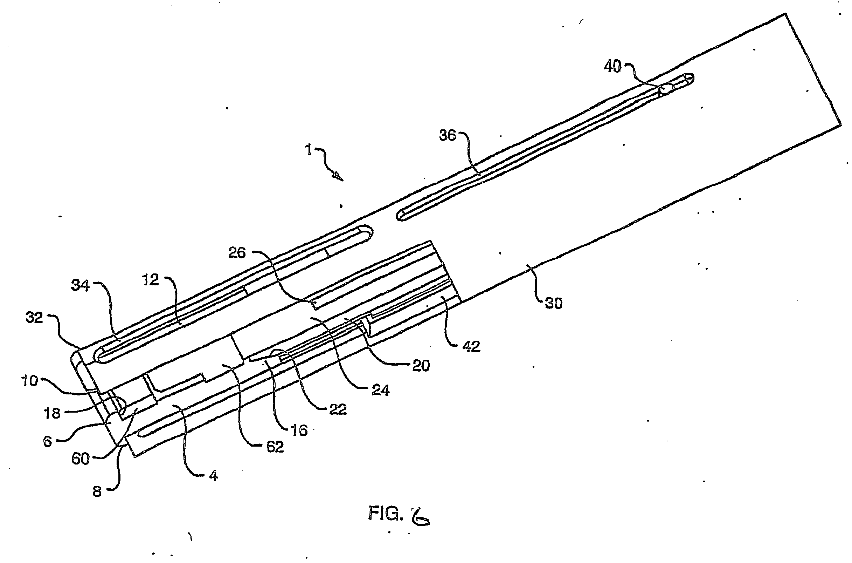

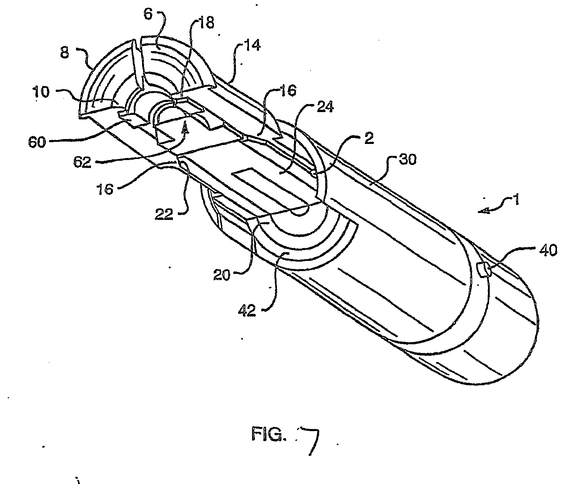

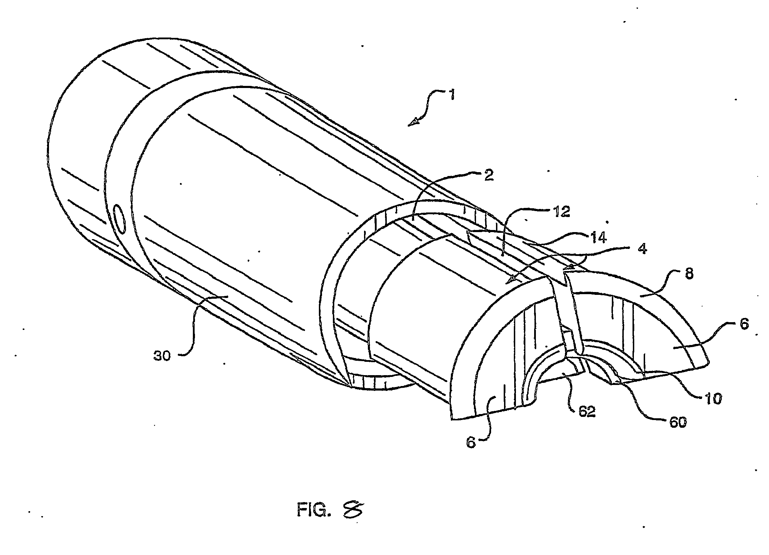

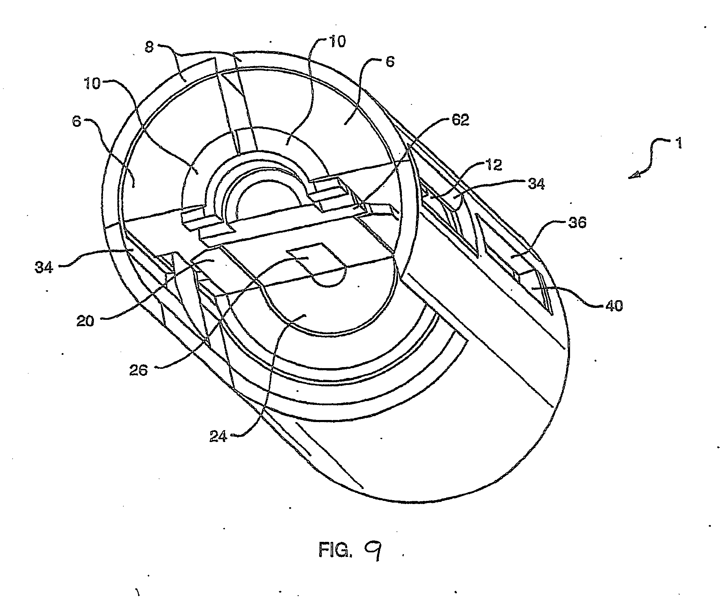

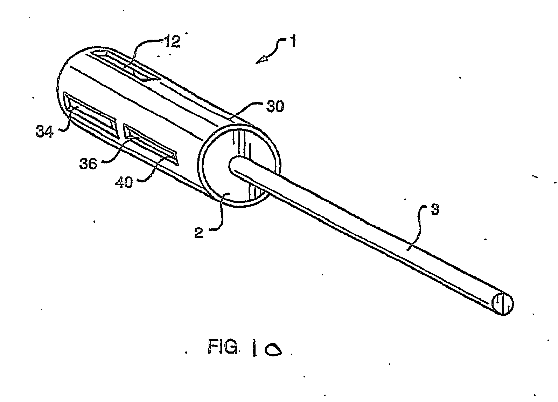

Referring to Figures 6 to 10, the distal end of a double pusher suture clip delivery and locking catheter is shown generally as 1. Catheter distal end 1 is comprised primarily of a collet cage 2 to which the other components of distal end 1 are attached. Collet cage 2 is essentially a cylinder with two or more collet fingers 4 extending distally from a distal end of collet cage 2. Extending radially inwardly from a distal end of each collet finger 4 is a collet finger flange 6 that functions as a stop to arrest distal advancement of a suture clip loaded into collet cage 2. The combination of the distal end of collet cage 2, collet fingers 4 and distal flanges 6 define a cage within which the components of a suture clip are releasably encapsulated for delivery to a sutured tissue site. The cage further functions to align the suture clip components for assembly.

In a preferred embodiment, collet finger flanges 6 have radiused outer distal edges 8 to minimize trauma to a patient and radiused inner distal edges 10 to ease loading of suture clip components. In a preferred embodiment, outer distal edges 8 extend radially outwardly beyond outer collet finger walls 14 to function as a stop for an outer sliding sleeve 30 described below. Inner proximal faces of collet finger flanges 6 are oriented to a longitudinal axis of collet fingers so that a plane occupied by flange proximal surfaces 18 forms an angle from about 90° to about 135° and preferably either 135° or 90° with 90° being the most preferred to maximize the stopping function.

Finger slots 12 are formed between and defined by collet fingers 4 and function as egress ports for sutures threaded through the components of a suture clip loaded into the collet cage. Preferably, collet fingers 4 are biased in an open position so that radial force need only be applied to move the fingers from an open, suture clip loading/releasing position to a closed, suture clip confining position. Alternatively, finger collets 4 can be biased in a closed position.

Formed on an inner wall and toward the proximal end of collet fingers 4 are ramps 16 that taper radially inwardly from proximal to distal ends. Ramps 16 function as cam surfaces that when engaged with a pusher, as described below, cause collet fingers 4 to open. The distance between a proximal face of collet finger flanges 6 and the most distal point of ramps 16 is set to accommodate at least one set of unassembled suture clip components. This distance can be modified to receive the components for multiple suture clips.

Situated within a hollow chamber defined by the inner walls of collet cage 2 is an outer pusher 20 that freely slides within collet cage 2. Pusher 20 is preferably a hollow cylinder. A distal end 22 of outer pusher 20 is formed with a taper on the outside wall of pusher 20 that preferably conforms to and mates with the angle formed by ramp 16. Distal advancement of outer pusher 20 engages pusher distal end 22 with ramp 16. As outer pusher 20 slides distally along ramp 16, collet finger 4 is forced open. Outer pusher 20 can be used with either a collet finger that is biased in a closed position or an open position.

Situated within the hollow chamber formed by the walls of outer pusher 20 is cylindrically shaped inner pusher 24. Inner pusher 24 slides freely within outer pusher 20. Inner pusher 24 is designed to engage a head of a suture plug situated in the collet cage. Distal advancement of inner pusher 24 engages the suture plug and drives the suture plug distally into a suture clip ring. Collet finger flanges 6 function as a stop for the suture ring so that the distal axial force applied causes engagement of the suture clip components.

An inner pusher bore 26 is formed in inner pusher 24 and extends from a point proximal to the distal end of inner pusher 24 proximally through and out a proximal end of inner pusher 24. Inner pusher bore 26 provides a chamber for receiving a wire (not shown) that is used to apply axial force to inner pusher 24. Use of a wire provides adequate force to accomplish suture clip assembly and allows for flexibility over the length of the catheter.

Situated in coaxial relationship with and freely sliding about collet cage 2 is outer sliding sleeve 30 that performs at least two functions; providing radial force against collet fingers 4 to maintain the fingers in a closed position during suture clip delivery to a tissue site to minimize potential trauma that could be caused by open collet fingers and providing a means to sever the tail ends of a suture that has been secured with a suture clip. When advanced distally, sliding sleeve 30 encompasses collet fingers 4 and restricts radial movement of the fingers regardless whether the fingers are biased in an open or closed position. In this position, sliding sleeve 30 prevents outer pusher 20 from prematurely opening the collect.

When proximally retracted, sliding sleeve 30 severs directly or cooperates with other components to sever suture material proximal to a cinched suture clip. In one embodiment, a distal end of suture slot 34 engages the suture and carries it toward a distal end of the body of collet cage 2. When the distal end of suture slot 34 travels past the distal end of the body of collet cage 2, the suture is severed. In another embodiment, sliding sleeve 34 interacts with a fixed cutter 42, described below, to sever the suture tail ends.

At least one suture slot 34 is formed toward a distal end 32 of sliding sleeve 30 to provide egress for excess suture material that typically extends beyond the orifice through which the catheter is inserted. It is important that finger slots 12 and suture slots 34 are at least partially aligned to allow a path for excess suture material to exit the suture clip delivery device. To accomplish alignment, an alignment slot 36 is formed preferably near a proximal end of sliding sleeve 30. An alignment pin 40 is affixed to collet cage 2 and dimensioned to freely slide within alignment slot 36. The length of alignment slot 36 limits the proximal and distal travel of sliding sleeve 30. Alignment pin 40 is positioned on collet cage 2 and alignment slot 36 is positioned in sliding sleeve 30 such that at least one finger slot 12 and suture slot 34 are aligned along their longitudinal axes. Preferably two diametrically opposed sets of finger slots 12 and suture slots 34 are provided to allow egress for each end of a suture.

An optional feature of the presently described embodiment is a fixed cutter 42. Cutter 42 is preferably a metallic ring formed about and affixed to collet 2 that has a distal edge 44 that is sufficiently sharp to sever suture material. The ring is used for embodiments that are preferably injection molded. However, the use of filled or engineered plastics can be used to obviate the need for fixed cutter 42.

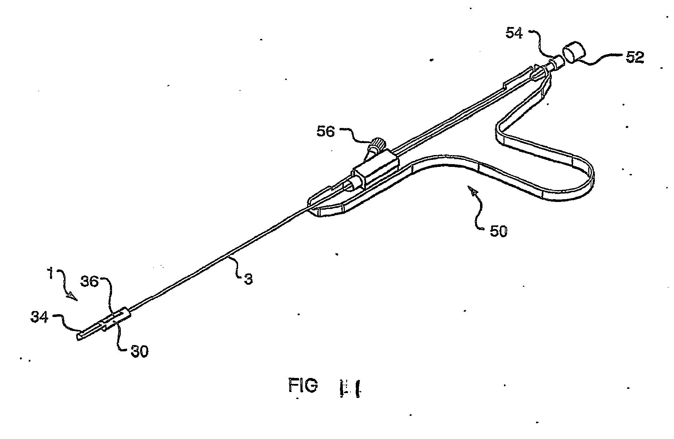

A pistol grip control handle, well known in the art, is used to manipulate the various sliding components of the catheter. As shown in Figure 11, pistol grip control 50 has three control surfaces for advancing and retracting the inner pusher 24, the outer pusher 20 and the outer sliding sleeve 30. A first control knob 52 and a second control knob 54 are coaxially arranged at a proximal end of pistol grip 50 and operate inner pusher 24 and outer pusher 20, respectively. A third control knob 56 extends from a top surface of, and in the distal end of, pistol grip 50. Third control knob 56 is connected to and operates outer sliding sleeve 30.

The first control knob 52 is connected to inner pusher 24 via a wire (not shown) that is preferably 0.76mm (0.030 inches) in diameter and that fits within and frictionally engages the walls defining inner pusher bore 26. The second control knob 54 is connected to outer pusher 20 via a first hypotube (not shown) coaxially arranged about the inner pusher wire and that is preferably 1.067mm (0.042 inches) in diameter. The pistol grip control 50 is attached to the catheter distal end 1 (the collect cage assembly) by a second hypotube 3 that is preferably 1.27mm (0.050 inches) in diameter and coaxially arranged about the first hypotube. The third control knob 54 is attached to sliding sleeve 30 via a third hypotube (not shown) that is preferably about 1.651mm (0.065 inches) in diameter and coaxially arranged about the second hypotube. Bushings between the hypotubes are provided in the collet cage assembly to seal the assembly and do not form a part of the invention. Preferably, the outer diameter of the catheter distal end 1 is 1.702mm (0.067 inches) when all the components are assembled. This ensures a wide application of use for the invention.

To load suture clip components, first control knob 52 and third control knob 56 are placed in proximally retracted positions. If collet fingers 4 are biased in an open position, second control knob 54 can also be placed in a proximally retracted position. Otherwise, second control knob 54 is placed in a distally advanced position to open collet fingers 4 by causing distal end 22 of outer pusher 20 to engage ramps 16.

With collet fingers 4 arranged in an open position, ends of a suture 80 that has been used to stitch tissue in the internal regions of an individual are threaded through a suture clip ring and into thread apertures formed in a suture clip plug. The suture ends are then fed through either the same or separate sets of finger slots 12 and suture slots 34 so that the ends of the suture are arranged external to the suture clip delivery catheter. The suture clip plug is then placed in a proximal end of the collet cage and the suture clip ring is placed in a distal end of the collet cage where the ring preferably engages at least one of the collet finger flanges 6 to prohibit distal travel of the ring. It is to be understood that the collet cage can be sized to accommodate a plurality of suture clip plugs that can be stacked for deployment into suture clip rings that must be loaded one per suture clipping procedure.

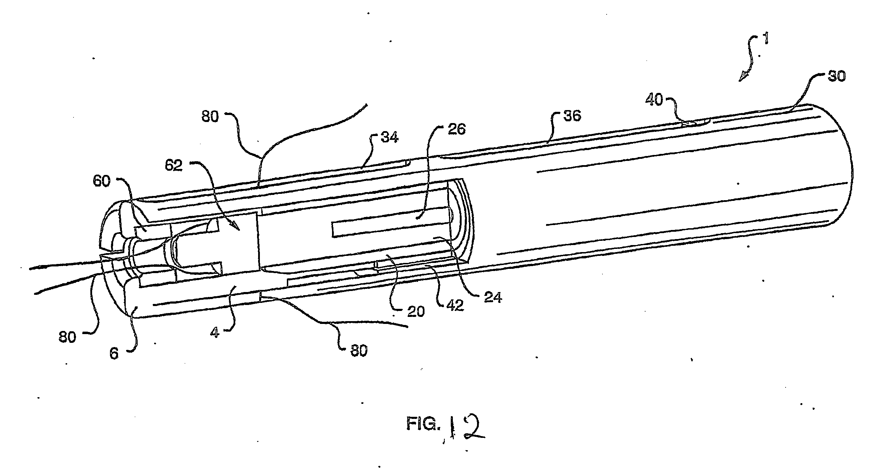

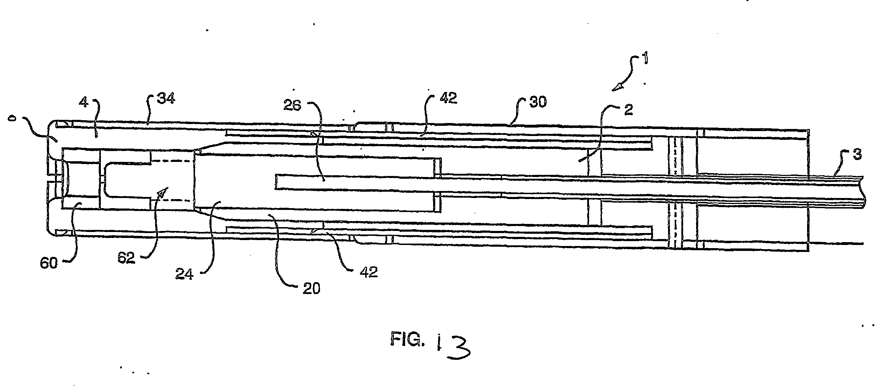

As shown in Figures 12 and 13, with the suture clip components loaded, second control knob 54 is retracted if previously advanced, and third control knob 56 is distally advanced so that outer sliding sleeve 30 engages and applies a radially constraining force to collet fingers 4 to maintain the fingers in a closed position during insertion of the catheter into a patient. In this configuration, catheter distal end 1 is advanced to the suture-clipping site in a patient.

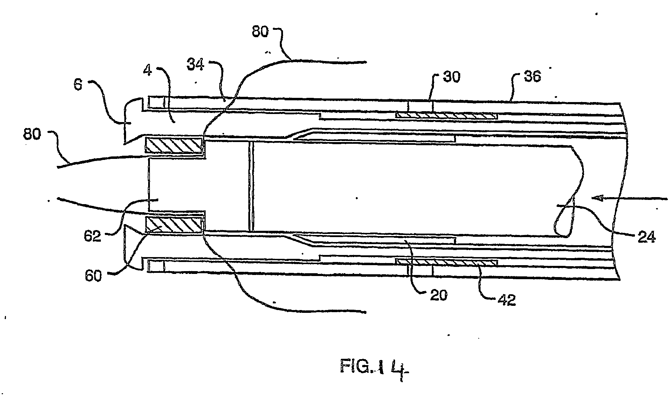

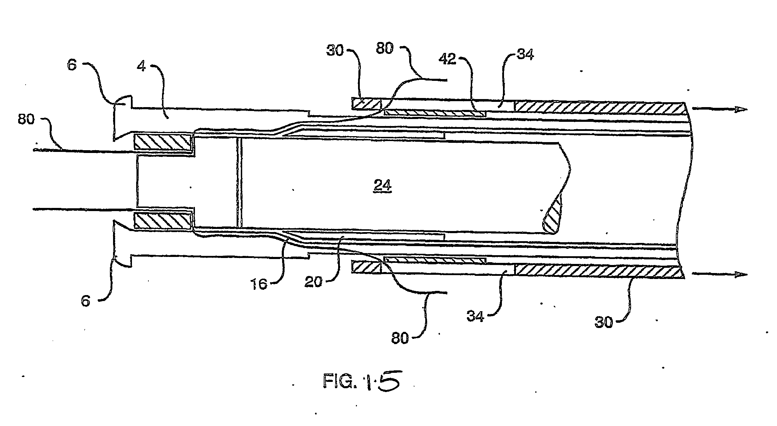

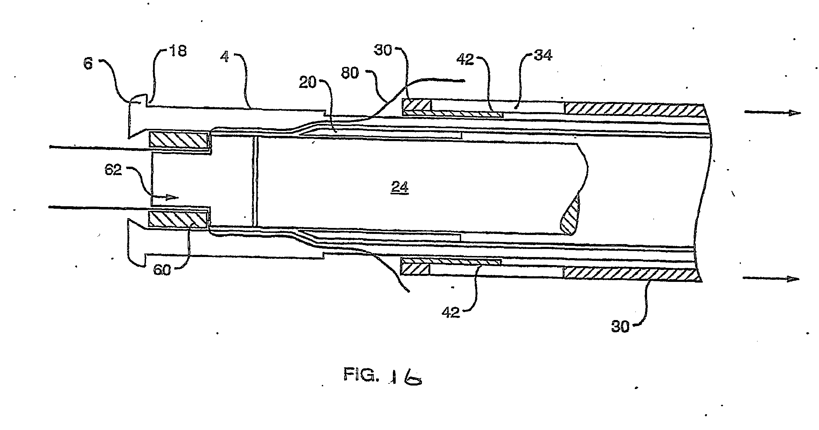

To operate, first control knob 52 is distally advanced so that inner pusher 24 engages the suture clip plug and forces a distal shaft of the plug into the suture clip ring. The frictional engagement of the suture clip plug to the suture clip ring captures the suture 80 via frictional engagement as shown in Figure 14. The advancement of inner pusher 24 also causes the suture clip components to be cinched in close proximity to the stitched tissue. Once the plug has been secured to the ring and the assembled suture clip cinched to the stitched tissue, third control knob 56 is retracted to release the radial force applied to the collet fingers 4 by sliding sleeve 30. The proximal retraction of sliding sleeve 30 also causes a distal end of suture slot 34 to engage suture 80 and carry it proximally toward either the sharp distal end of the collet cage 2 or the distal edge 44 of fixed cutter 42 as shown in Figure 15. When the distal end of suture slot 34 passes proximally beyond either the distal end of the collet body or distal edge 44, the suture is severed proximal to the assembled clip as shown in Figure 16. The severed ends of the suture can then be pulled out of the individual.

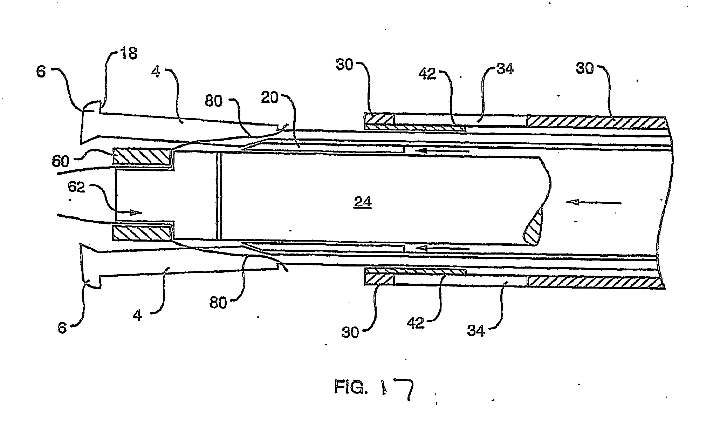

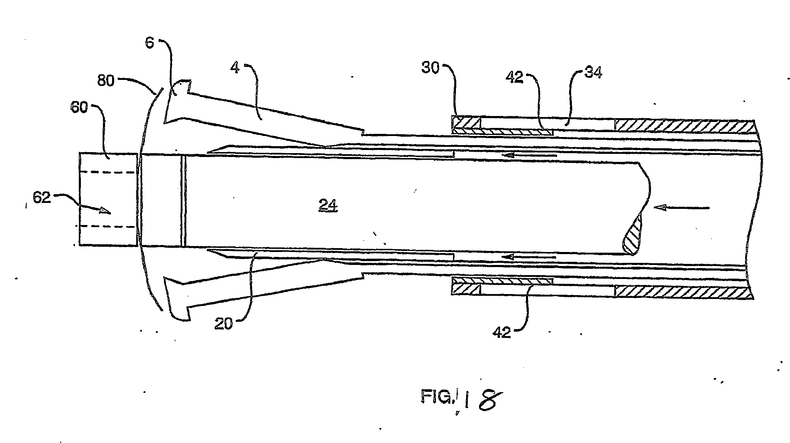

If collet fingers 4 are biased in a closed position, second control knob 54 is distally advanced to ramp open the collet fingers to release the assembled plug as shown in Figure 17. Once the suture clip has been released as shown in Figure 18, second control knob 54 is retracted and third control knob 56 is advanced to place collet fingers 4 in a closed position to reduce the potential for trauma when the catheter is removed from the individual.

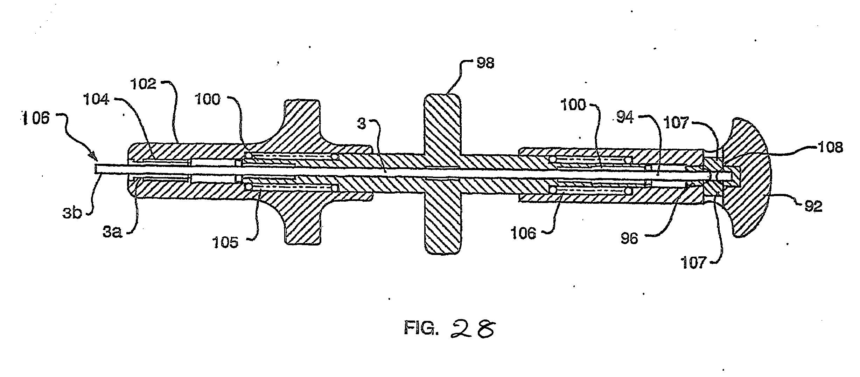

In an alternate embodiment shown in Figure 28, control handle 90 can be provided with springs to ensure that the proper orientation of the moving parts is maintained before and after the delivery and cinching of the suture clip components. First return spring 105 provides an axial force that maintains pusher rod 94 in a proximal position while second return spring 106 provides an axial force that maintains sliding cutter sleeve 30 in a distal position. The springs are selected to provide enough tension to maintain the pusher rod and sliding cutter sleeve in starting positions when advancing and retracting the suture clip delivery device. This provides desired protection to an endoscope that could potentially be damaged if collet fingers 4 where allowed to remain in radially extended positions when advancing or retracting the delivery device in the endoscope. The springs are further selected so that the manual forces needed to overcome the springs to advance pusher rod 94 and to retract sliding cutter sleeve 30 are within acceptable ranges.

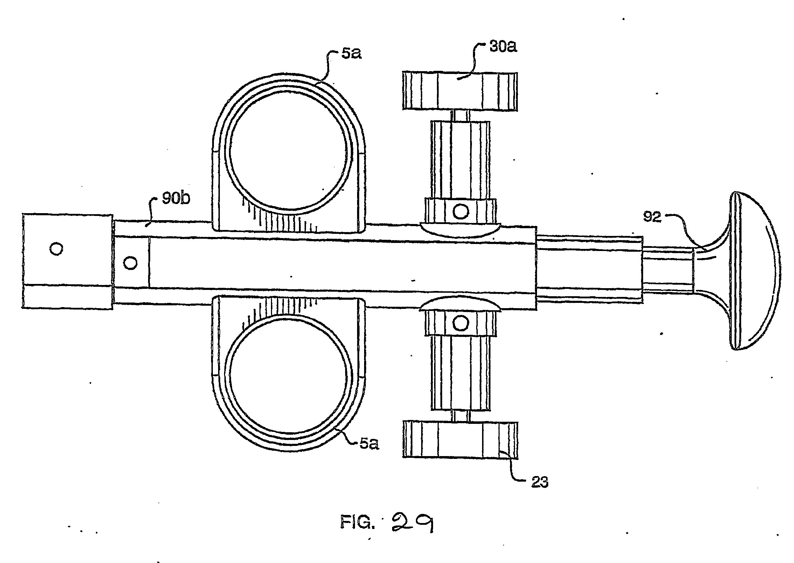

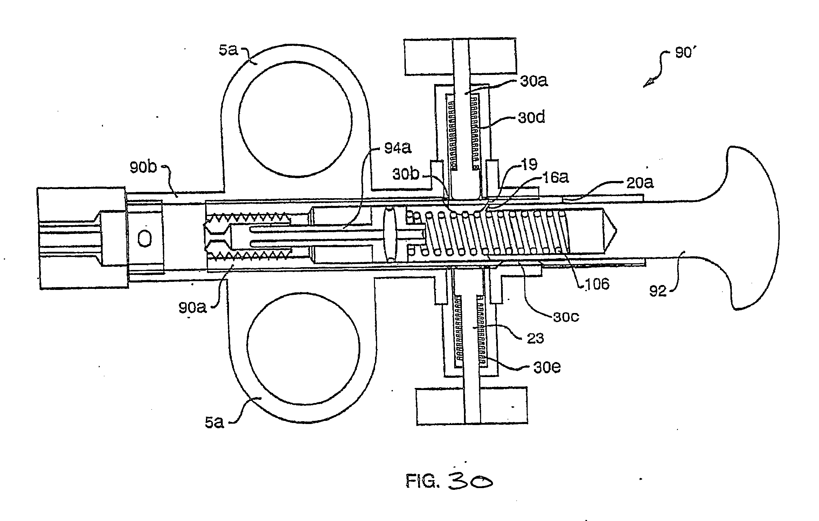

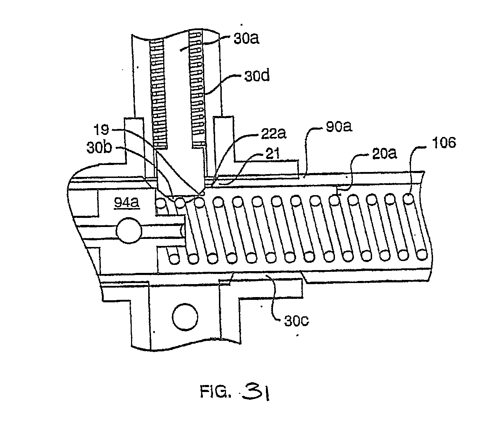

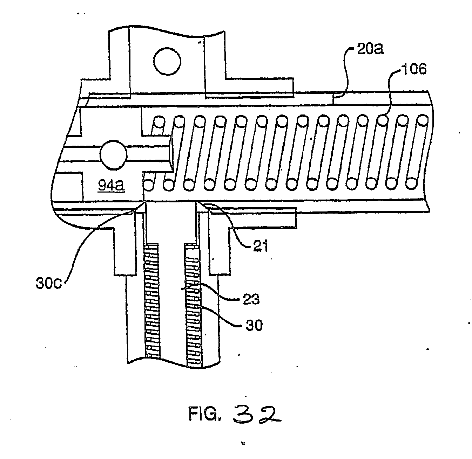

In a further embodiment shown in Figures 29 to 32, a control handle 90' provides a means of cinching the suture clip components, releasing the cinched suture clip and severing the suture tails with a single action. In this embodiment, a handle main body 90a extends distally and terminates as collet cage 2. An outer handle sleeve 90b is provided about main body 90a, extends distally and terminates as sliding sleeve 30. To secure plug 62 into ring 60, a distally directed force is applied to cinch handle 92' to overcome the force of second return spring 106 which has an axial pre-bias load in tension. Distal advancement of cinch handle 92 relative to handle main body 90a causes second return spring 106 to engage a proximal end of a ramrod 94a and cause the distal advancement of ramrod 94a into a proximal end of pusher 94. Distal advancement of pusher 94 causes engagement and distal advancement of plug 62. Second return spring 106 is of sufficient resiliency to resist compression while moving ramrod 94a distally to effectuate the insertion of plug 62 into ring 60.

A restraining force to hold outer handle sleeve 90b and sliding sleeve 30 in a stationary position relative to advancing ramrod 94a and cinch handle 92 is provided by a user's fingers engaged in finger rings 5a, mounted to a proximal end of outer handle sleeve 90b. Spring plunger 30a provided at the proximal end of outer handle sleeve 90b, engages cutout 30b formed in handle main body 90a to temporarily lock outer handle sleeve 90b and main body 90a together during distal advancement of ramrod 94a. Spring plunger 30a, biased in a radially extended position by a plunger spring 30d, remains extended and in a locked position with respect to main body 90a. Plunger spring 30d have an axial pre-bias load in tension. Spring plunger 30a may be compressed only when the distal angular face 16a of cinch handle 92 has advanced distally to a point of tangency with angular face 19 of spring plungers 30a. Distal movement of the distal angular face 16 drives spring plunger 30a in a direction perpendicular to the central axis, a distance equivalent to the thickness 20a of the wall of cinch handle 92. At this point, the restraining forces provided by the user's fingers (in a proximal direction), acting on main body 90a through finger rings 5a, have been restrained. The locking action of spring plunger 30a is an interaction of a radial surface 22a with pusher face 21. Alignment of angular face 19 with angular pusher face 21 results in continued radial outward motion of spring plunger 30a with the continued distal motion of main body 90a.

Main body 90a continues distally with the application of palm pressure on cinch handle 92 and the relative opposite motion of finger rings 5a (proximally). Suture 80 (not shown), lying in a path of sliding sleeve 30 is severed with the proximal travel of sliding sleeve 30. Collet fingers 4 are now unrestrained and assume their biased open position releasing cinched plug 62 and ring 60.

In the most retracted stage of sliding sleeve 30 and finger rings 5a, a second spring plunger 23 drops into a second cutout 30c. Second spring plunger 23 is maintained in a compressed state via second plunger spring 30e. Second plunger spring 30e has an axial pre-bias load in compression. Second spring plunger 23 remains in second cutout 30c only with the application of a force by the user's thumb on the most outward end of second spring plunger 23. This temporary locking feature is provided to maintain sliding sleeve 30 in its most proximal position to allow for loading of plug 62 and ring 60 as described in detail herein. Release of the holding pressure automatically releases sliding sleeve 30 to affect a closure of the delivery system.

To operate this embodiment of the suture clip delivery system, following the loading of a suture clip assembly and the threading of sutures through ring 60 described below, the entire delivery device is advanced through the endoscope to the sutured tissue site. Next, cinch handle 92 is advanced toward collet cage handle 98 to cinch plug 62 into ring 60 so that suture 80 is captured between the mating surfaces of a plug distal shaft 68 and the inner walls of ring 60. Following completion of the cinching step, sliding sleeve handle 102 is retracted toward collet cage handle 98 to simultaneously sever the unused ends of suture 80 and to allow collet fingers 4 to spring open and release the suture clip assembly. To complete the procedure, the suture clip delivery system is partially retracted so that the suture clip is clear of the delivery system distal end. Prior to full retraction, sliding sleeve handle 102 is advanced to move collet fingers 4 into a closed position to allow for the full retraction of the suture clip delivery system out of the endoscope. With the spring embodiment of the handle, release of the users' grip on sliding sleeve handle 102 allows first return spring 105 to relax thereby advancing sliding sleeve handle 102 and sliding cutter sleeve 30 to a preferably fully advanced position.

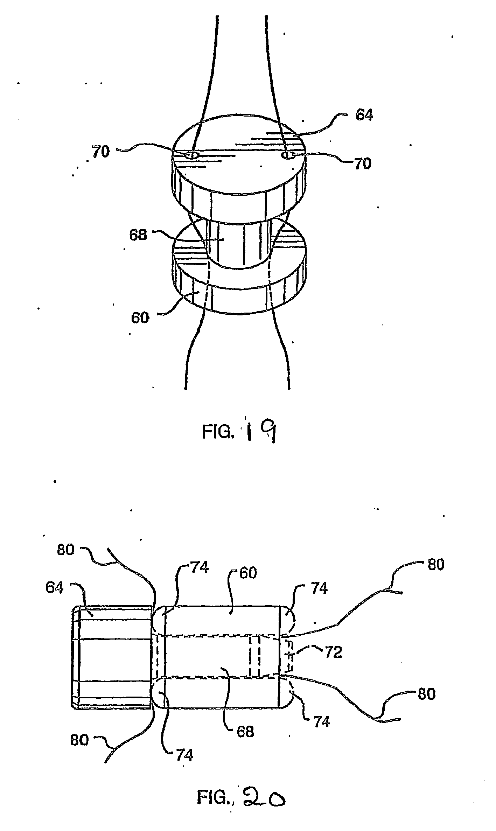

The suture clips used with the suture clip delivery catheter and/or endoscopic system described herein, have plugs with heads. In another embodiment as shown in Figure 19, plug 62 has distal shaft 68 that is adapted to frictionally engage the inner walls of ring 60 and a head 64 that has preferably two suture receiving bores 70 formed toward the perimeter of the head 64. This configuration provides a suture clip plug that decreases the effort needed to advance the plug over the ends of a suture before loading into the suture clip delivery device.

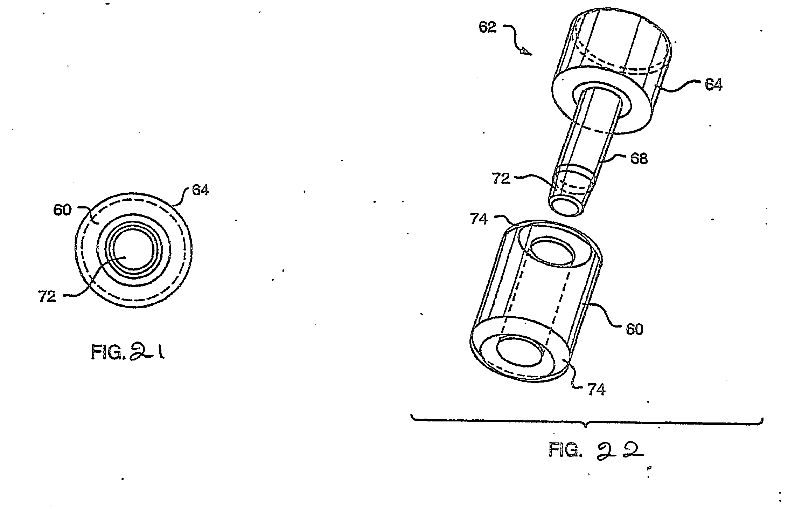

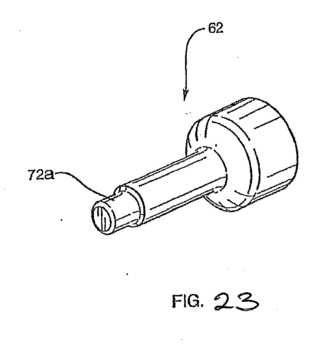

In a preferred embodiment shown in Figures 20 to 22, the inner and outer walls of ring 60 are radiused at the ends 74 to facilitate the insertion of distal shaft 68 into ring 60. More importantly, the radiused ends provide strain relief for the sutures 80 when compressed between the plug 62 and ring 60. To further facilitate insertion, distal shaft 68 is formed with a tapered distal tip 72. Alternatively, a reduced neck distal tip 72a can be employed as shown in Figure 23. Although interlocking features can be incorporated into the plug and clip components, it has been found that such features can lead to suture fraying, premature fracturing and failure. Use of tapered or reduced tipped plugs 62 allow for a preferred gradual reduction in compression. It has been found that suture capture is optimized and suture destruction is minimized when a distally decreasing taper leading end is used on plug 62 with a proximally increasing diameter of plug 62 to exceed the net diameter of the suture 80 in a captured state plus the diameter of plug 62 by at least 0.051mm (.002 inches). The preferred materials used to make plug 62 and ring 60 are polyetherether ketone (PEEK) 450G, PEEK-Optima™ LT or polyethylene terephthalate (PET).

As previously stated, suture 80 is held via friction between plug 62 and ring 60. For example, a 0.229mm (.009 inch) thick suture is captured in a 0.076mm (.003 inch) gap between plug 62 and ring 60 so that the suture will remain captured with the application of a three pound load. The aforementioned radiused and tapered surfaces of plug 62 and ring 60 provide enough strain relief to prevent damage to suture 80 when subjected to the three-pound load.

A further suture clip embodiment employs a head with a proximal surface that is convex. Preferably, the distal end of inner pusher 24 is provided with a concave surface to matingly engage the convex surface of plug head. This configuration aids with the axial alignment of the ring and plug during distal advancement and engagement.

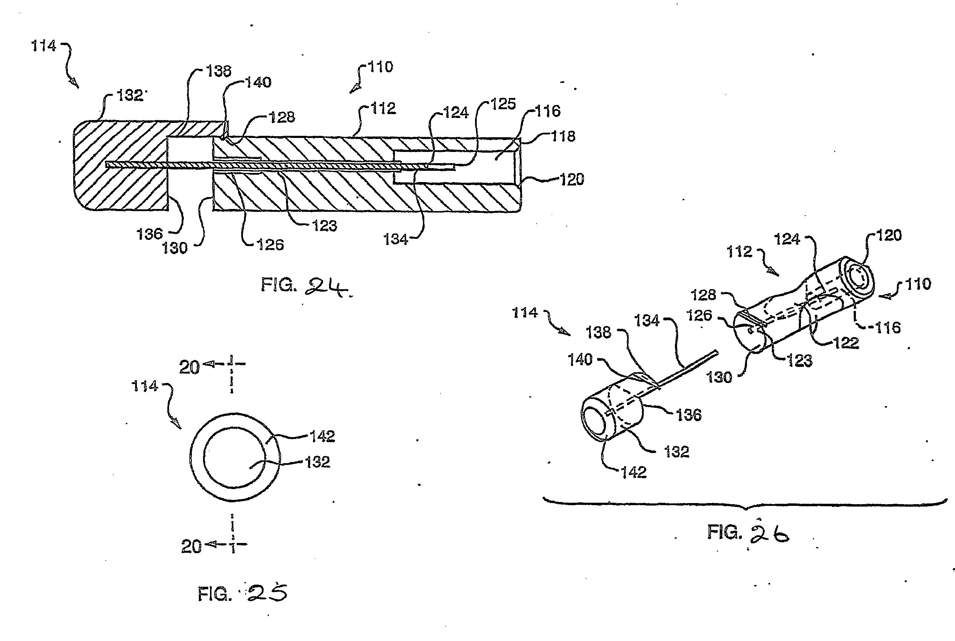

To load the suture clip assembly into collet cage 2, a suture clip loader device 110 is provided as shown in Figures 24 to 26. Loader device 110 comprises a loader housing 112 that is substantially cylindrical in shape and a plunger 114 that is also substantially cylindrical in shape. Loader housing 112 has a collet cavity 116 formed in a first end 118 that is dimensioned to receive collet cage 2. Collet cavity 116 has a cavity end 120 tapered to facilitate insertion of collet cage 2. Formed on diametrically opposed surfaces of a sidewall of loader housing 112 are finger grooves 122 that provide a stable holding surface for manipulating loader housing 112 during a suture clip loading procedure. A loader hypotube 124 is secured with an adhesive 126 (preferably cyanoacrylate Loctite® 4013 or 4014) to a loader hypotube shoulder 123 and a bore formed along the longitudinal axis of loader housing 112. Loader hypotube 124 extends from a second end 130 of loader housing 112 to at least partially within collet cavity 116 and is dimensioned to receive at a distal hypotube end 125, the tapered distal tip 72 of plug 62. A transverse groove 128 is formed proximal to second end 130 of loader housing 112. The function of groove 128 will be discussed below.

Plunger 114 comprises a plunger head 132 and a plunger rod 134 secured via friction fit or adhesive in a bore formed along a longitudinal axis of plunger head 132. Plunger rod 134 is sized to freely slide within hypotube 124. Extending from a first plunger end 136 of plunger head 132 is tab 138. A top surface of tab 138 is contoured like, and coplanar with, the outer surface of plunger head 132 while a bottom surface is substantially flat and oriented substantially perpendicular to first plunger end 136. Extending downwardly from a distal end of tab 138 is a flange 140 that is shaped to conform to the shape of groove 128. Optionally, plunger head 132 can be formed with a radiused second plunger end 142 for ease of handling.

The cross-sectional diameter of plunger head 132 is sized so that the distance between the bottom surface of tab 138 and the most distant point on the cross-sectional circumference of plunger head 132 is substantially equal to the cross-sectional diameter of loader housing 112. When plunger rod 134 is inserted into hypotube 124, the bottom surface of tab 138 rides along the outer surface of loader housing 112. Because flange 140 extends below the bottom surface of tab 138, advancement of plunger 114 toward loader housing 112 results in flange 140 engaging groove 128 and temporarily locking in the distance between plunger 114 and loader housing 112. In this orientation, a distal end of plunger rod 134 does not reach distal hypotube end 125 so that plug 62 can be received in hypotube end 125.

The preferred materials for the suture clip loader device 110 components are as follows. Plunger head 132 is made from Delrin® and is preferably white. Plunger rod 134 is made from 304V stainless steel. Loader hypotube shoulder 123 is made from 304 stainless steel. Loader housing is made from polycarbonate GE Lean® 104-1111 and is preferably clear to allow for an unobstructed view of hypotube end 125. Hypotube 124 is made from polyetherether ketone (PEEK) 450G.

The operation of suture clip loader device 110 is as follows. The process begins by placing ring 60 over hypotube 124. Next, the distal tip 72 of plug 62 is inserted into hypotube end 125 until it is snugly secured to hypotube 124. At this point, plunger 114 is secured to loader housing 112 via the engagement of flange 140 to groove 128.

With collet cage 2 extending from the distal end of an endoscope, sliding sleeve 30 is retracted to allow collet fingers 4 to spring into an open position. Collet cage 2 is advanced over hypotube 124 and ring 60 until the distal end of collet cage 2 engages a bottom of collet cavity 116. Once the suture clip components have been correctly encapsulated by collet fingers 4, force is applied to plunger 114 to overcome the locking engagement of flange 140 and groove 128. This enables plunger rod 134 to be advanced through hypotube 124 to contact and eject plug 62 into collet cage 2. Proper ejection is assured when first plunger end 136 contacts second end 130 of loader housing 112. To capture ring 60 within collet cage 2, sliding sleeve 30 is advanced to move collet fingers 4 into a closed position about ring 60 and hypotube 124. Collet finger flanges 6 engage a distal face of ring 60 so that retraction of collet cage 2 from hypotube 124 results in ring 60 being retracted off hypotube 124 and secured within the distal end of collet cage 2.

It is important that plug 62 is maintained in the proximal end of collet cage 2 and that ring 60 is maintained in the distal end of collet cage 2. This is required to enable suture 80 to be threaded through ring 60 prior to cinching and final deployment of the suture clip assembly.

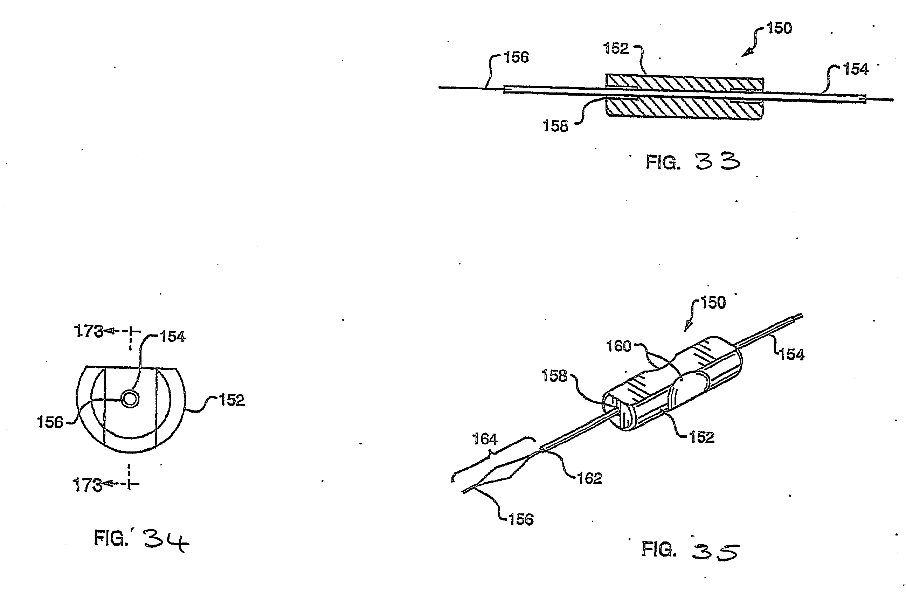

The next step in the procedure is to thread sutures previously secured to tissue through ring 60. To perform this procedure, a threader or suture loop tool 150 is used. As shown in FIGS. 33-35, threader 150 comprises a threader housing 152 that is substantially cylindrical in shape and preferably made from polycarbonate GE Lean® 104-1111. Formed on diametrically opposed surfaces of a sidewall of threader housing 152 are threader finger grooves 160 that provide a stable holding surface for manipulating threader housing 152 during a suture threading procedure. Affixed to a bore formed along the longitudinal axis of threader housing 152 is suture loop hypotube 154. An adhesive 158 such as cyanoacrylate Loctite® 4013 (clear) is used to secure suture loop hypotube 154 (preferably made from 304 stainless steel), to threader housing 152. A suture loop 156 preferably made of two thin 304v stainless steel wires is secured inside suture loop hypotube 154 and extends beyond a distal end 162 of suture loop hypotube 154. A suture loop distal end 164 is formed into a diamond shape by overlapping the stainless steel wires. Preferably, a tip of the suture loop distal end is formed by overlapping the wires at least twice.

To thread suture 80 through ring 60, suture loop 156 of threader 150 is inserted into suture slot 34 of sliding sleeve 30, advanced through ring 60 and through the distal end of collet cage 2. Suture 80 is inserted into the diamond-shaped suture loop distal end so that it engages and preferably becomes entangled with the intertwined distal tip of suture loop distal end 164. To thread suture 80 through ring 60, threader 150 is retracted out of suture slot 34. Suture 80 is then removed from threader 150. The suture clip assembly is now ready for cinching and deployment as described above.

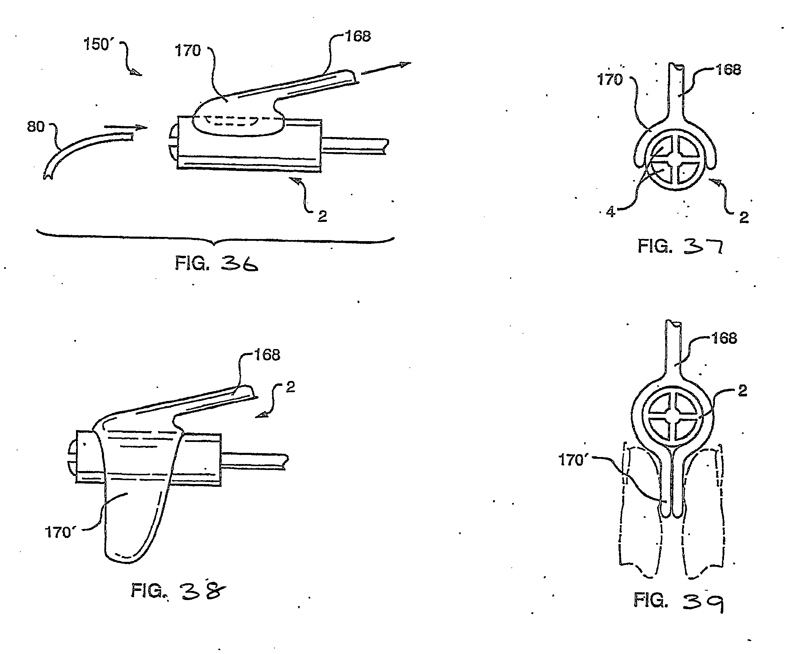

In an alternate embodiment, threader 150 comprises a vacuum-actuated nozzle 150' made from a pliable polymeric or elastomeric material. As shown in Figure 36, a nozzle head 170 is formed at the end of a vacuum hose 168 and adapted to conform to the contoured shape of collet cage 2. A proximal end of vacuum hose 168 (not shown) is attached to a vacuum source (not shown). In a further embodiment shown in Figures 38 and 39, nozzle head 170 is formed with nozzle ears 170" that preferably extend about and beyond collet cage 2 to enhance the ease with which the nozzle head can be secured against suture slot 34, again with either finger or forceps pressure.

To operate nozzle 150', nozzle head 170 is maintained against suture slot 34 with either finger pressure or pressure exerted with forceps while a vacuum is applied. The vacuum draws suture 80 through ring 60 and out of collet cage 2 via suture slot 34. The vacuum is then released, nozzle head 170 is removed from collet cage 2 and suture 80 is grasped and pulled a desired amount through ring 60 and collet cage 2.

It should be understood that the foregoing description of the invention is intended merely to be illustrative thereof and that other modifications and embodiments may be apparent to those who are skilled in the art. Having thus described the invention what we desire to claim and secure by letters patent is:

## Claims

1. A suture clip delivery catheter comprising:
a collet (2) having a body and a plurality of collet fingers (4) extending from a distal end of the body; the collet fingers (4) being movable from a closed position to retain a suture clip and an open position to release a suture clip;
a tube (3) for connecting the collet (2) to a control handle;
an outer sleeve (30) situated coaxially about the collet (2), the outer sleeve (30) being freely slidable along a longitudinal axis from a first position to constrain the collet fingers (4) in the closed position to a second position to permit the collet fingers (4) to move to the open position;
a first pusher (24) situated coaxially within the collet (2) that freely slides along a longitudinal axis, the first pusher (24) configured to engage a suture clip to secure a suture,
**characterised by** a second pusher (20) situated coaxially about the first pusher (24) and coaxially within the collet (2) that freely slides along the longitudinal axis, the second pusher (20) freely slides within the collet (2) and is configured to cause the collet fingers (4) to move to the open position.

2. The catheter of claim 1, wherein said outer sleeve (30) is configured to sever a suture upon longitudinal movement of the outer sleeve (30) from the first position to the second position.

3. The catheter of claim 2, comprising a suture slot (34) in the outer sleeve (30) configured so that a distal end of said suture slot (34) engages a suture and carries it toward a distal end of the collet (2) to be severed.

4. The catheter of claim 3, wherein the suture slot (34) is configured so that a suture is severed when said distal end of said suture slot (34) travels past the distal end of the collet (2).

5. The catheter of claim 3, comprising a cutter (42) affixed to the collet (2) to sever a suture.

6. The catheter of any preceding claim, wherein the plurality of collet fingers (4) further comprise flanges (6) extended radially inwardly from a distal end of each of the plurality of collet fingers (4).

7. The catheter of claim 6, wherein at least one of the plurality of collet fingers (4) has a ramp (16) formed on an inside wall that tapers radially inwardly from a proximal to a distal end such that the combination of the collet fingers (4), ramp (16) and flanges (6) define a collet cage (2).

8. The catheter of claim 7, wherein a distal end of the second pusher (20) is tapered to matingly engage the ramp (16) when the second pusher (20) is distally advanced.

9. The catheter of any preceding claim, wherein the flanges (6) are radiused on outer and inner distal edges.

10. The catheter of any preceding claim, wherein each of the plurality of collet fingers (4) has sidewalls that define finger slots (12).

11. The catheter of claim 10, wherein each of the plurality of collet fingers (9) has sidewalls that define finger slots (12).

12. The catheter of claim 11, wherein the sleeve (30) has further portions defining an alignment slot (36) situated proximal to a distal end (32) of the sleeve (30).

13. The catheter of claim 12, wherein the collet has an alignment pin (40) formed on an outer collet wall adapted to engage and slide within the alignment slot (36) such that at least one of the finger slots (12) radially aligns with the suture slot (34).

14. The catheter of claim 13, wherein the at least one finger slot (12) longitudinally aligns with at least a portion of the suture slot (34).

15. The catheter of claim 6, wherein a proximal face of the flanges (6) forms an inclusive angle with a longitudinal axis of an adjoined collet finger (4) of from about 90° to about 135°.

## Patentansprüche

1. Wundklammerabgabekatheter, der Folgendes umfasst:
eine Aufnahme (2) mit einem Körper und mehreren Aufnahmefingern (4), die sich von einem distalen Ende des Körpers erstrecken; wobei die Aufnahmefinger (4) aus einer geschlossenen Stellung zum Zurückhalten einer Wundklammer und einer geöffneten Stellung zum Freigeben einer Wundklammer bewegbar sind;
ein Rohr (3) zum Verbinden der Aufnahme (2) mit einem Steuergriff;
eine Außenhülle (30), die sich koaxial um die Aufnahme (2) herum befindet, wobei die Außenhülle (30) entlang einer Längsachse aus einer ersten Stellung zum Halten der Aufnahmefinger (4) in der geschlossenen Stellung in eine zweite Stellung zum Zulassen, dass die Aufnahmefinger (4) sich in die geöffnete Stellung bewegen, frei gleitbar ist;
eine erste Schubvorrichtung (24), die sich koaxial in der Aufnahme (2) befindet und die entlang einer Längsachse frei gleitet, wobei die erste Schubvorrichtung (24) dazu konfiguriert ist, eine Wundklammer in Eingriff zu nehmen, um einen Nahtfaden zu sichern,
**gekennzeichnet durch** eine zweite Schubvorrichtung (20), die sich koaxial um die erste Schubvorrichtung (24) herum und koaxial in der Aufnahme (2) befindet und die entlang der Längsachse frei gleitet, wobei die zweite Schubvorrichtung (20) in der Aufnahme (2) frei gleitet und dazu konfiguriert ist zu bewirken, dass die Aufnahmefinger (4) sich in die geöffnete Stellung bewegen.

2. Katheter nach Anspruch 1, wobei die Außenhülle (30) dazu konfiguriert ist, einen Nahtfaden bei einer Längsbewegung der Außenhülle (30) aus der ersten Stellung in die zweite Stellung zu durchtrennen.

3. Katheter nach Anspruch 2, der einen Nahtfadenschlitz (34) in der Außenhülle (30) umfasst, der derart konfiguriert ist, dass ein distales Ende des Nahtfadenschlitzes (34) einen Nahtfaden in Eingriff nimmt und ihn in Richtung eines distalen Endes der Aufnahme (2) trägt, um durchtrennt zu werden.

4. Katheter nach Anspruch 3, wobei der Nahtfadenschlitz (34) derart konfiguriert ist, dass ein Nahtfaden durchtrennt wird, wenn das distale Ende des Nahtfadenschlitzes (34) das distale Ende der Aufnahme (2) passiert.

5. Katheter nach Anspruch 3, der eine Schneidvorrichtung (42) umfasst, die an der Aufnahme (2) angebracht ist, um einen Nahtfaden zu durchtrennen.

6. Katheter nach einem vorhergehenden Anspruch, wobei die mehreren Aufnahmefinger (4) weiterhin Flansche (6) umfassen, die sich radial nach innen von einem distalen Ende jedes der mehreren Aufnahmefinger (4) erstrecken.

7. Katheter nach Anspruch 6, wobei mindestens einer der mehreren Aufnahmefinger (4) eine Rampe (16) aufweist, die auf einer Innenwand ausgebildet ist und die sich radial nach innen von einem proximalen Ende zu einem distalen Ende verjüngt, so dass die Kombination der Aufnahmefinger (4), der Rampe (16) und der Flansche (6) einen Aufnahmekäfig (2) definiert.

8. Katheter nach Anspruch 7, wobei ein distales Ende der zweiten Schubvorrichtung (20) verjüngt ist, um die Rampe (16) zusammenpassend in Eingriff zu nehmen, wenn die zweite Schubvorrichtung (20) distal vorgeschoben wird.

9. Katheter nach einem vorhergehenden Anspruch, wobei die Flansche (6) auf äußeren und inneren distalen Kanten gerundet sind.

10. Katheter nach einem vorhergehenden Anspruch, wobei jeder der mehreren Aufnahmefinger (4) Seitenwände aufweist, die Fingerschlitze (12) definieren.

11. Katheter nach Anspruch 10, wobei jeder der mehreren Aufnahmefinger (9) Seitenwände aufweist, die Fingerschlitze (12) definieren.

12. Katheter nach Anspruch 11, wobei die Hülle (30) weitere Abschnitte aufweist, die einen Ausrichtungsschlitz (36) definieren, der sich proximal zu einem distalen Ende (32) der Hülle (30) befindet.

13. Katheter nach Anspruch 12, wobei die Aufnahme einen Ausrichtungsstift (40) aufweist, der auf einer Aufnahmeaußenwand ausgebildet ist und dazu eingerichtet ist, den Ausrichtungsschlitz (36) in Eingriff zu nehmen und in diesem zu gleiten, so dass mindestens einer der Fingerschlitze (12) sich radial auf den Nahtfadenschlitz (34) ausrichtet.

14. Katheter nach Anspruch 13, wobei der mindestens eine Fingerschlitz (12) sich in Längsrichtung auf mindestens einen Abschnitt des Nahtfadenschlitzes (34) ausrichtet.

15. Katheter nach Anspruch 6, wobei eine proximale Fläche der Flansche (6) einen einschließenden Winkel mit einer Längsachse eines angrenzenden Aufnahmefingers (4) von etwa 90° bis etwa 135° bildet.

## Revendications

1. Cathéter d'application de clip de suture, comprenant :
une douille de serrage (2) possédant un corps et une pluralité de doigts de douille de serrage (4) qui s'étendent depuis une extrémité distale du corps, lesdits doigts de douille de serrage (4) pouvant être déplacés d'une position fermée dans laquelle ils retiennent un clip de suture jusqu'à une position ouverte dans laquelle ils libèrent le clip de suture ;
un tube (3) servant à relier la douille de serrage (2) à une poignée de commande ;
un manchon extérieur (30) disposé coaxialement autour de la douille de serrage (2), ledit manchon extérieur (30) pouvant coulisser librement le long d'un axe longitudinal depuis une première position dans laquelle il contraint les doigts de douille de serrage (4) dans la position fermée jusqu'à une seconde position dans laquelle il permet aux doigts de douille de serrage (4) de se déplacer en position ouverte ;
un premier poussoir (24), disposé coaxialement à l'intérieur de la douille de serrage (2), qui coulisse librement le long d'un axe longitudinal, ledit premier poussoir (24) étant configuré pour venir en prise avec un clip de suture afin de réaliser une suture,
**caractérisé par** un second poussoir (20), disposé coaxialement autour du premier poussoir (24) et coaxialement à l'intérieur de la douille de serrage (2), qui coulisse librement le long de l'axe longitudinal, ledit second poussoir (20) coulissant librement à l'intérieur de la douille de serrage (2) et étant configuré pour forcer les doigts de douille de serrage (4) à se déplacer dans la position ouverte.

2. Cathéter selon la revendication 1, dans lequel ledit manchon extérieur (30) est configuré pour couper une suture lors du déplacement longitudinal du manchon extérieur (30) de la première position à la seconde position.

3. Cathéter selon la revendication 2, comprenant dans le manchon extérieur (30) une fente de suture (34) configurée de telle façon qu'une extrémité distale de ladite fente de suture (34) vient en prise avec une suture et l'amène en direction d'une extrémité distale de la douille de serrage (2) afin de la couper.

4. Cathéter selon la revendication 3, dans lequel la fente de suture (34) est configurée de telle façon qu'une suture est coupée lorsque ladite extrémité distale de ladite fente de suture (34) passe au-delà de l'extrémité distale de la douille de serrage (2).

5. Cathéter selon la revendication 3, comprenant une lame (42) fixée à la douille de serrage (2) afin de couper une suture.

6. Cathéter selon l'une quelconque des revendications précédentes, dans lequel la pluralité de doigts de douille de serrage (4) comprend en outre des collerettes (6) qui s'étendent radialement vers l'intérieur depuis une extrémité distale de chaque doigt de la pluralité de doigts de douille de serrage (4).

7. Cathéter selon la revendication 6, dans lequel l'un au moins des doigts de le pluralité de doigts de douille de serrage (4) possède une rampe (16) formée sur une paroi intérieure qui s'amincit radialement vers l'intérieur d'une extrémité proximale à une extrémité distale de telle façon que la combinaison des doigts de douille de serrage (4), de la rampe (16) et des collerettes (6) définit une cage de douille de serrage (2).

8. Cathéter selon la revendication 7, dans lequel une extrémité distale du second poussoir (20) présente un amincissement correspondant pour venir en prise avec la rampe (16) lorsque le second poussoir (20) avance en direction distale.

9. Cathéter selon l'une quelconque des revendications précédentes, dans lequel les collerettes (6) possèdent des arêtes distales extérieures et intérieures rayonnées.

10. Cathéter selon l'une quelconque des revendications précédentes, dans lequel chaque doigt de la pluralité de doigts de douille de serrage (4) possède des parois latérales qui définissent des fentes de doigts (12).

11. Cathéter selon la revendication 10, dans lequel chaque doigt de la pluralité de doigts de douille de serrage (9) possède des parois latérales qui définissent des fentes de doigts (12).

12. Cathéter selon la revendication 11, dans lequel le manchon (30) possède d'autres portions qui définissent une fente d'alignement (36) située en position proximale par rapport à une extrémité distale (32) du manchon (30).

13. Cathéter selon la revendication 12, dans lequel la douille de serrage possède un pion d'alignement (40), formé sur une paroi extérieure de la douille de serrage, qui est adapté pour s'engager et coulisser dans la fente d'alignement (36) de telle façon que l'une au moins des fentes de doigts (12) s'aligne radialement avec la fente de suture (34).

14. Cathéter selon la revendication 13, dans lequel la ou les fentes de doigts (12) s'alignent longitudinalement avec au moins une portion de la fente de suture (34).

15. Cathéter selon la revendication 6, dans lequel une face proximale des collerettes (6) forme avec un axe longitudinal d'un doigt de douille de serrage (4) adjacent un angle inclus d'environ 90° à environ 135°.
